# EUROPÄISCHE PATENTSCHRIFT

(11) **EP 0 951 907 B1**
(45) Veröffentlichungstag und Bekanntmachung des Hinweises auf die Patenterteilung: **05.03.2003**
(21) Anmeldenummer: 99106438.7
(22) Anmeldetag: 29.03.1999
(51) Int. Cl.: A61K 31/505, A61P 3/00

(54) **Verwendung von Moxonidin als Thermogenese stimulierend wirksames Arzneimittel**
Use of moxonidine as a thermogenesis stimulating agent
Utilisation de la moxonidine en tant qu'agent stimulateur de la thermogenèse

(30) Priorität: 06.04.1998 DE 19815411
(43) Veröffentlichungstag der Anmeldung: 27.10.1999
(73) Patentinhaber: Solvay Pharmaceuticals GmbH, 30173 Hannover (DE)
(72) Erfinder: Williams, Gareth, Prof. Dr., Wirral L60 0DT (GB); Bing, Chen, Dr., Liverpool L25 SHN (GB); Kaan, Elbert, Dr., 30938 Grossburgwedel (DE); Ziegler, Dieter, Dr., 30966 Hemmingen (DE)
(74) Vertreter: Gosmann, Martin

(56) Entgegenhaltungen:
- WO-A-96/26728
- WO-A-97/46241
- DE-A- 3 904 795
- BING, C. ET AL: "Anorexic and weight loss effect of moxonidine is accompanied by inhibition of hypothalamic NPY and mRNA in obese Zucker rats." EUROPEAN JOURNAL OF CLINICAL INVESTIGATION, Bd. 28, Nr. suppl.1, Mai 1998 (1998-05), Seite A32 XP002113730
- ERNSBERGER, P. ET AL: "Sympathetic nervous system in salt-sensitive and obese hypertension: amelioration of multiple abnormalities by a central sympatholytic agent." CARDIOVASC DRUGS THER, JUN 1996, 10 SUPPL 1 P275-82, XP002113731 UNITED STATES
- ERNSBERGER P ET AL: "The I1-imidazoline receptor: from binding site to therapeutic target in cardiovascular disease." J HYPERTENS SUPPL, JAN 1997, 15 (1) PS9-23, XP002113732 ENGLAND
- HENRIKSEN EJ ET AL: "Antihypertensive agent moxonidine enhances muscle glucose transport in insulin-resistant rats." HYPERTENSION, DEC 1997, 30 (6) P1560-5, XP002113733 UNITED STATES
- FRIEDMAN JE ET AL: "Anti-hyperglycemic activity of moxonidine: metabolic and molecular effects in obese spontaneously hypertensive rats." BLOOD PRESSURE, Bd. 7, Nr. suppl. 3, 1998, Seiten 32-39, XP002113734 NORWAY
- ROSEN P ET AL: "Experimental benefit of moxonidine on glucose metabolism and insulin secretion in the fructose-fed rat." J HYPERTENS SUPPL, JAN 1997, 15 (1) PS31-8, XP002113735 ENGLAND
- WELLMAN PJ ET AL: "Effects of the alpha 1-adrenergic agonist cirazoline on locomotion and brown adipose tissue thermogenesis in the rat." LIFE SCI, 1992, 50 (23) P1745-53, XP002113736 ENGLAND
- NISOLI E ET AL: "SR 58611A: a novel thermogenic beta-adrenoceptor agonist." EUR J PHARMACOL, JUL 1 1994, 259 (2) P181-6, XP002113737 NETHERLANDS
- SANTTI, E. ET AL: "Potentiation of the anti-obesity effect of the selective beta 3-adrenoceptor agonist BRL 35135 in obese Zucker rats by exercise." BR J PHARMACOL, DEC 1994, 113 (4) P1231-6, XP002113738 ENGLAND
- SAVONTAUS E ET AL: "Anti-obesity effect of MPV-1743 A III, a novel imidazoline derivative, in genetic obesity." EUR J PHARMACOL, JUN 11 1997, 328 (2-3) P207-15, XP002113739 NETHERLANDS
- BING, CHEN ET AL: "The effect of moxonidine on feeding and body fat in obese Zucker rats: Role of hypothalamic NPY neurones." BRITISH JOURNAL OF PHARMACOLOGY, 1999, XP002113740

## Beschreibung

Die vorliegende Erfindung betrifft die Verwendung von 4-Chlor-5-[(4,5-dihydro-lH-imidazol-2-yl)-amino]-6-methoxy-2-methylpyrimidin (= Moxonidin) und dessen physiologisch verträglichen Säureadditionssalzen zur Behandlung und/oder Prophylaxe von Thermogenese-Störungen und/oder zur Stimulierung (Erhöhung) der Thermogenese bei Personen mit anomal niedrigem Energieumsatz ("Verbrennung") und/oder bei Personen deren Thermogenese während oder im Anschluß an eine gewichtsreduzierende Diät gemindert ist, und zur Herstellung von für diese Behandlung und/oder Stimulierung geeigneten Arzneimitteln.

In einigen wissenschaftlichen Veröffentlichungen wird Moxonidin als ein zentral wirksamer selektiver I₁-Imidazolin Rezeptor Agonist beschrieben, der die Gewichtsreduzierung bei fettleibigen hypertensiven Syndromen fördert: So z.B. von Ernsberger et al. (Cardiovascular Drugs Ther., June 1996, 10 Suppl. 1, 275-282), unter dem Titel "Sympathetic Nervous System in Salt-Sensitive and Obese Hypertension: Amelioration of Multiple Abnormalities by a Central Sympatholytic Agent". Henriksen et al. beschreiben, wie das antihypertensive Agens Moxonidin den Glucosetransport im Muskel Insulin-resistenter Ratten erhöht (Hypertension, Dec. 1997, 30 (6), 1560-1565, "Antihypertensive Agent Moxonidine Enhances Muscle Glucose Transport in Insulin-Resistant Rats"). Friedman et al. diskutieren die anti-hyperglykämische Wirkung von Moxonidin, insbesondere die metabolischen und molekularen Effekte in fettleibigen, spontan antihypertensiven Ratten (Blood Pressure, Bd. 7, Suppl. 3, 1998, 32-39, Anti-hyperglycemic Activity of Moxonidine: Metabolic and Molecular Effects in Obese Spontaneously Hypertensive Rats"). Ferner ist im Stand der Technik beschrieben, daß Moxonidin zur Behandlung von Patienten mit kardiovaskulärer Störung und Insulinresistenz verwendet werden könnte. So diskutieren Ernsberger et al. diese Eigenschaft im Zusammenhang mit den I₁-Imidazolin Rezeptor unter dem Aspekt "von der Bindungsstelle zu einem therapeutischen Ziel bei kardiovaskulären Krankheiten" (J. Hypertens. Suppl., Jan. 1997, 15 (1), S9-S23, "The I₁-Imidazoline receptor:from binding site to therapeutic Target in cardiovascular disease"). Rösen et al. beschreiben die experimentellen Vorteile von Moxonidin auf den Metabolismus und die Insulin-Sekretion in Fructose-gefütterten Ratten (J. Hypertens. Suppl., Jan. 1997, 15 (1), S31-S38, Experimental benefit of moxonidine on glucose metabolism and insulin secretion in the fructose-fed rat"). In den vorstehend zitierten Dokumenten des Standes der Technik finden sich jedoch keine Hinweise darauf, daß Moxonidin zur Herstellung von pharmazeutischen Zubereitungen zur Förderung der Thermogenese, beziehungsweise zur Behandlung oder Prophylaxe thermogenetischer Unterfunktionen verwendet werden könnte.

Der Erfindung liegt die Aufgabe zugrunde, neue pharmazeutische Zubereitungen zu entwickeln, welche geeignet sind zur Behandlung von auf einer ungenügenden Thermogenese beruhenden Störungen des Energiehaushaltes und welche eine Förderung der Thermogenese insbesondere ohne cardiovascular-stimulierende Nebenwirkungen bewirken.

Erfindungsgemäß werden zur Herstellung von pharmazeutischen Zubereitungen zur Förderung der Thermogenese und/oder zur Behandlung und/oder Prophylaxe von thermogenetischer Unterfunktion, insbesondere ohne Erhöhung von Blutdruck und Herzfrequenz, 4-Chlor-5-[(4,5-dihydro-1H-imidazol-2-yl)-amino]-6-methoxy-2-methylpyrimidin der Formel I 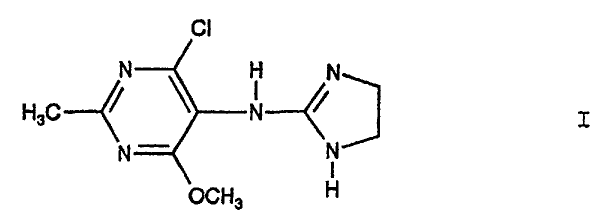 und dessen physiologisch verträgliche Säureadditionssalze verwendet.

Als physiologisch verträgliche Säureadditionssalze des Moxonidins eignen sich Salze mit anorganischen Säuren, beispielsweise Halogenwasserstoffsäuren, oder mit organischen Säuren, beispielsweise niederen aliphatischen Mono- oder Dicarbonsäuren wie Essigsäure, Fumarsäure oder Weinsäure oder aromatischen Carbonsäuren wie z. B. Salicylsäure.

Die erfindungsgemäß eingesetzten Verbindungen fallen unter den Umfang von in der deutschen Offenlegungsschrift Nr. 28 49 537 beschriebenen 5-[(2-Imidazolin-2-yl)-amino]-pyrimidin-Derivaten mit blutdrucksenkenden Eigenschaften, und sind aus dieser Patentanmeldung bekannt. Moxonidinhaltige pharmazeutische Zubereitungen sind als Antihypertensiva unter den Markennamen Physiotens^{R} im Handel erhältlich und werden medizinisch als Antihypertensivum eingesetzt. Die Verbindungen können auf an sich bekannte Weise nach den in der vorgenannten Offenlegungsschrift beschriebenen Verfahren oder analog zu diesen Verfahren hergestellt werden.

Es wurde nun überraschenderweise gefunden, daß Moxonidin und seine physiologisch verträglichen Säureadditionssalze eine die Thermogenese fördernde Wirkung am Menschen und größeren Säugetieren besitzen und zur Behandlung von mit unzureichender Thermogenese einhergehenden Stoffwechselstörungen des Energiehaushaltes geeignet sind.

Der Energiehaushalt eines Patienten kann dadurch gestört sein, daß er an einer thermogenetischen Unterfunktion leidet und sein metabolischer Grundumsatz von Natur aus so niedrig ist, daß die aufgenommenen Nahrungsmengen nicht vollständig thermogenetisch verwertet und in Verbrennungsenergie überführt werden können, sondern teilweise in Fett umgewandelt in Fettzellen gespeichert werden, und auf Grund der ungenügenden thermogenetischen Aktivität die metabolische Fettverwertung unzureichend ist.

Ein adaptives Abfallen der Thermogenese tritt z. B. im Rahmen von gewichtsreduzierenden Diätkuren und im Anschluß daran auf, da der Körper bei zu geringer Nahrungsaufnahme reflektorisch die thermogenetische Aktivität einschränkt, sozusagen auf Sparflamme schaltet, und auch bei Wiederaufnahme einer normal ausreichenden Nahrungszufuhr die Thermogenese weiterhin unerwünscht vermindert bleibt und die während der Diät abgebauten Fettdepots wieder aufgefüllt werden.. Durch Reduzierung des Energieverbrauches vermindert der Körper somit effektiv die Wirksamkeit der gewichtsreduzierenden Medikation oder Diät.

Eine Thermogenese-Stimulierung wird daher zur Verhinderung einer reflektorischen Thermogenese-Hemmung während und nach einer gewichtsreduzierenden Diät und/oder Medikation (z. B. mit appetitzügelnden Medikamenten) benötigt, um eine anschließende Gewichtswiederzunahme zu vermeiden.

Behandlungsbedürftige Störungen der Thermogenese, welche zu Störungen der Thermoregulierung mit daraus resultierender Hypothermie (= Absinken der Körpertemperatur auf abnormal niedrige Werte) führen können, können auch auftreten als Folge von Unterkühlung (z. B. zu langes der Kälte Ausgesetztsein bzw. zu langes Eintauchen in kaltes Wasser) und/oder als Begleiterscheinung von diversen Krankheiten.

Auf Grund seiner Thermogenese fördernden Wirkung ist Moxonidin geeignet, die thermogenetische Aktivität der Verbrennungsenergie produzierenden Gewebe und den Metabolismus in den Fettzellen zu stimulieren und die thermogenetische Grundumsatzrate zu steigern.

Es sind verschiedene andere thermogenetische Substanzen bekannt, agieren aber peripher auf das wärmeerzeugende Gewebe, durch Aktivierung der noradrenergen Rezeptoren. Da ähnliche Rezeptoren auch am Herzen und an Arterien vorhanden sind, üben diese peripher wirkenden Substanzen vielfach auch eine stimulierende Wirkung auf Herz-Kreislauffunktionen aus und können so zu Blutdrucksteigerungen führen.

Das erfindungsgemäß zur Thermogenesestimulation eingesetzte Moxonidin zeichnet sich durch ein überraschend andersartiges Wirkprofil aus, in welchem zentral wirksame Thermogenese fördernde Eigenschaften mit blutdrucksenkenden Eigenschaften gekoppelt sind. Moxonidin bewirkt eine Stimulierung des Verbrennungsenergie erzeugenden Gewebes und gleichzeitig eine Dämpfung des cardiovasculären Geschehens. Dieses günstige Wirkprofil der thermogenesefördernden Wirkung von Moxonidin ist darauf zurückzuführen, daß diese überwiegend durch die zentrale Wirkung von Moxonidin auf die die Thermogenese regulierenden Zentren im Hypothalamus ausgelöst wird.

Da Übergewicht oftmals mit erhöhtem Blutdruck und/oder Herzbeschwerden vergesellschaftet ist, ist ein Pharmakon, das einer thermogenetischen Unterfunktion entgegenwirkt und den thermogenetischen Energieverbrauch erhöht, ohne blutdrucksteigernde Nebenwirkungen zu zeigen, ja sogar zusätzlich blutdrucksenkende Eigenschaften besitzt, besonders vorteilhaft.

Zur erfindungsgemäßen Behandlung von Zuständen gestörter und/oder unerwünscht verminderter Thermogenese können Moxonidin und seine physiologisch verträglichen Säureadditionssalze in üblichen pharmazeutischen Zubereitungen oral, intravenös oder auch transdermal verabreicht werden.

So können die Thermogenese fördernd wirksame Mengen der Verbindungen erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Supporitorien. Diese festen Präparate können pharmazeutisch übliche anorganische und/oder organische Trägerstoffe wie z. B. Milchzucker, Talkum oder Stärke neben pharmazeutisch üblichen Hilfsmitteln, beispielsweise Gleitmitteln oder Tablettensprengmitteln, enthalten. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die Thermogenese fördernden Wirkungen von Moxonidin können in Standard Tests zur Bestimmung von pharmakologischen Indikatoren für die Wirkung von Moxonidin auf die Thermogenese beeinflussende Faktoren in Zuckerratten nachgewiesen werden.

Es wurde gezeigt, daß in fetten Zuckerratten der Gehalt von uncoupling protein-1 (UCP-1) mRNA wesentlich reduziert war im Vergleich zu dem ihrer schlanken Artgenossen. Dies spiegelt eine beeinträchtigte thermogenetische Funktion, welche in diesem Tiermodell wahrscheinlich auf einer zentralen genetisch bedingten Fehlregulierung des sympathischen Nervensystems zurückzuführen ist, wieder.

### Testbeschreibung

9 schlanke und 18 fette 9 Wochen alte Zuckerratten erhielten während 21 Tagen Moxonidin in einer Tagesdosis von 3 mg/kg per os. Eine gleiche Anzahl Kontrolltiere erhielt nur Placebo. Den Tieren stand Futter unbegrenzt zur Verfügung.

Es wurde der Neuropeptide Y (= NPY) mRNA Gehalt im Hypothalamusgewebe bestimmt. NPY ist ein die Fettleibigkeit auf Grund von potenten appetitstimulierenden und antithermogenetischen Wirkungen förderndes Neuropeptid. Im Hypothalamus der fetten Ratten waren die NPY mRNA Spiegel bei den mit Moxonidin behandelten Tieren um 40 % im Vergleich zu den Kontrolltieren reduziert. Bei den schlanken Tieren wurde jedoch keine signifikante Reduzierung der NPY mRNA Spiegel beobachtet. Diese Daten sind ein Indiz dafür daß Moxonidin eine zentrale Hemmwirkung auf die NPY-Synthese im Arcuate Nucleus (ARC) des Hypothalamus hat; diese Inhibierung von NPY wird aus den Wirkungen des Peptides zur Aktivierung des energieverbrauchenden Gewebes und zur Erhöhung der thermogenetischen Aktivität im Fettgewebe z. B. im "Brown Adipose Tissue" (= BAT) abgelesen.

Ferner wurden als Indiz für die thermogenetische Aktivität im BAT die UCP-1 mRNA-Spiegel im BAT durch Messung nach der Nothern Blotting Analysenmethode bestimmt. Moxonidin bewirkt bei fetten und bei schlanken Tieren in etwa eine Verdoppelung der UCP-1-Gen Expression im Vergleich zu den Kontrolltieren.

Der Versuch mit fetten Tieren wurde unter pair-fed-Bedingungen (den mit Moxonidin behandelten Tieren stand Futter unbegrenzt zur Verfügung und die Kontrolltiere erhielten nur eine der von den Moxonidin-behandelten Tieren verzehrten Menge entsprechende Futtermenge) wiederholt, um den Einfluß unterschiedlicher Futteraufnahme zwischen der Kontrolltiergruppe und der Moxonidin-behandelten Tiergruppe auf die untersuchten Parameter auszuschließen.

In diesem Experiment zeigte sich sogar eine signifikante Verdreifachung der UCP-1-Genexpression bei den Moxonidin-behandelten Tieren im Vergleich zur pair-fed-Kontrollgruppe.

Diese Versuchsergebnisse sind ein deutlicher Hinweis auf eine nach Moxonidingabe verstärkte Thermogenese.

Die vorstehenden Testergebnisse sind ein Indiz für eine zentrale, Thermogenese fördernde Wirkung von Moxonidin.

Die vorstehenden Versuchsergebnisse zeigen, daß Moxonidin und seine Säureadditionssalze bei gestörter bzw. unerwünscht verminderter Thermogenese eine Thermogenese fördernde Wirkung ausüben und dadurch eine Erhöhung des Energieumsatzes bewirken, ohne jedoch einen normalen Energieumsatz zu beeinträchtigen. Moxonidin und seine Säureadditionssalze sind deshalb zur Behandlung von thermogenetischer Unterfunktion und/oder von gestörter und/oder unerwünscht verminderter Thermogenese geeignet. Die zu verwendeten Dosen können individuell verschieden sein und variieren naturgemäß je nach Art des zu behandelnden Zustandes und der Applikationsform. Im allgemeinen liegen die Tagesdosen zur Behandlung von Zuständen gestörter und/oder unerwünscht verminderter Thermogenese beim Menschen bei oraler Applikation im Bereich von 0,05 bis 5 mg, vorzugsweise 0,2 bis 0,8 mg.

Das folgende Beispiel soll die Herstellung einer zur Behandlung von unzureichender Thermogenese geeigneten Moxonidin enthaltenden pharmazeutischen Zubereitung näher erläutern.

### Beispiel 1:

### Moxonidin-haltige filmüberzogene Tabletten

### Zusammensetzung:

| Tablettenkerne: | |
|---|---|
| Moxonidin | 0,020 Teile |
| Lactose | 9,580 Teile |
| Povidone USP | 0,070 Teile |
| Crospovidone USP | 0,300 Teile |
| Magnesiumstearat | 0,030 Teile |
| (Wasser | 0,750 Teile) |
| Gesamtfeststoff | 10,000 Teile |

| Filmüberzug: | |
|---|---|
| Hydroxypropylmethylcellulose | 0,156 Teile |
| 30%ige wäßrige Ethylcellulose-Dispersion | 0,480 Teile |
| (= Feststoff) | (0,144) Teile |
| Polyethylenglycol 6000 | 0,030 Teile |
| Titandioxid | 0,150 Teile |
| Talc | 0,1197 Teile |
| Rotes Eisenoxid | 0,0003 Teile |
| (Wasser | 3,864 Teile) |
| Gesamtfeststoff | 0,600 Teile |
| Gesamtmenge an Filmüberzugssuspension | 4,800 Teile |

Zum Überziehen von 10.000 Tablettenkerne zu je 100 mg Gewicht werden 4,8 kg der vorstehenden Filmüberzugssuspension eingesetzt.

### Tablettenkernherstellung:

Das Moxonidin und die Lactose wurden gemischt. Die Mischung wurde mit einer Lösung des Bindemittels Povidone in Wasser durchfeuchtet, gut durchgeknetet und das erhaltene Produkt wurde auf Horden ausgebreitet und bei einer Temperatur von ca. 50 °C bis zu einem Feuchtigkeitsgehalt von höchstens 0,5 % getrocknet. Das getrocknete Produkt wurde durch ein 0,75 mm-Sieb (Frewitt-Maschine) passiert. Nach dem Vermischen des erhaltenen Granulates mit Crospovidone und Magnesiumstearat wurden daraus Tablettenkerne mit einem Gewicht von 100 mg gepreßt, so daß jeder Tablettenkern 20 mg Wirkstoff enthielt.

### Herstellung der Filmüberzugssuspension:

Die Hydroxypropylmethylcellulose und das Polyethylenglycol 6000 wurden in einem Teil des Wassers gelöst. Zu dieser Lösung wurde eine Suspension von Talc, Titandioxid und Eisenoxid in dem übrigen Wasser unter Rühren zugefügt. Die erhaltene Suspension wurde unter leichtem Rühren mit der 30%igen wäßrigen Ethylcellulose-Dispersion verdünnt.

### Filmüberziehen der Tablettenkerne:

Die Filmüberzugssuspension wurde auf die Tablettenkerne in einer Befilmungsapparatur gesprüht, während warme Luft von ca. 70 °C die Tablettenkerne auf eine Temperatur von ca. 45 °C erwärmte. Anschließend wurden die filmüberzogenen Tabletten 16 Stunden bei einer Temperatur von ca. 45 °C getrocknet.

## Patentansprüche

1. Verwendung von 4-Chlor-5-[(4,5-dihydro-1H-imidazol-2-yl)-amino]-6-methoxy-2-methylpyrimidin der Formel I 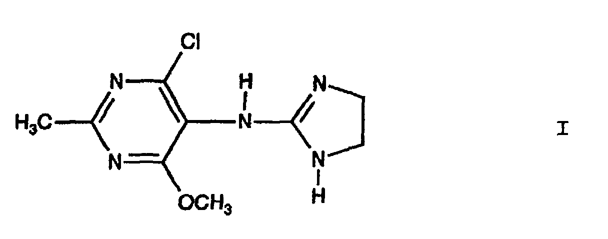 und dessen physiologisch verträglichen Säureadditionssalzen zur Herstellung von pharmazeutischen Zubereitungen zur Förderung der Thermogenese und/oder Behandlung und/oder Prophylaxe thermogenetischer Unterfunktion.

## Claims

1. The use of 4-chloro-5-[(4,5-dihydro-1H-imidazol-2-yl)-amino]-6-methoxy-2-methylpyrimidine of Formula I 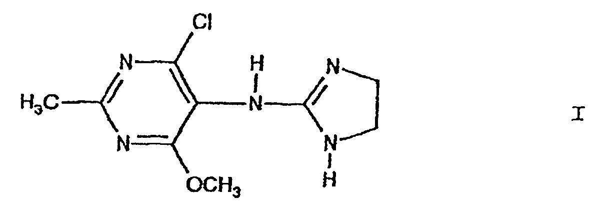 and the physiologically compatible acid addition salts thereof for the preparation of pharmaceutical preparations for promoting the thermogenesis and/or treatment and/or prophylaxis of thermogenic hypofunction.

## Revendications

1. Utilisation de 4-chloro-5-[(4,5-dihydro-1H-imidazol-2-yl)-amine]-6-méthoxy-2-méthylpyrimidine de formule I 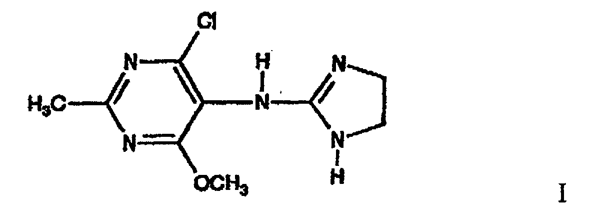 et de ses sels d'addition acides physiologiquement compatibles pour fabriquer des préparations pharmaceutiques permettant de favoriser la thermogenèse et/ou à des fins de traitement et/ou de prophylaxie d'une hypofonction de la thermogenèse.
